# EUROPÄISCHE PATENTSCHRIFT

(11) **EP 0 922 693 B1**
(45) Veröffentlichungstag und Bekanntmachung des Hinweises auf die Patenterteilung: **24.10.2001**
(21) Anmeldenummer: 98123151.7
(22) Anmeldetag: 04.12.1998
(51) Int. Cl.: C07C 63/10

(54) **Verfahren zur Herstellung von 3-Chlor-4-fluorbenzoylchlorid**
Process for the preparation of 3-chloro-4-fluorobenzoyl chloride
Procédé de préparation du chlorure de l'acide 3-chloro-4-fluorobenzoique

(30) Priorität: 12.12.1997 DE 19755298
(43) Veröffentlichungstag der Anmeldung: 16.06.1999
(73) Patentinhaber: Clariant GmbH, 65929 Frankfurt am Main (DE)
(72) Erfinder: Pfirmann, Ralf, Dr., 64347 Griesheim (DE); Wessel, Thomas, Dr., 60386 Frankfurt (DE)

(56) Entgegenhaltungen:
- DE-A- 2 914 915

## Beschreibung

Die vorliegende Erfindung betrifft ein vorteilhaftes Verfahren zur Herstellung von 3-Chlor-4-fluorbenzoylchlorid.

3-Chlor-4-fluorbenzoylchlorid und seine Derivate stellen wichtige Vorprodukte für die Herstellung von Pharmazeutika und Pflanzenschutzmitteln dar (J. Med. Chem. 1997, Vol. 40, 35 - 43, DE 29 14 915 A1).

3-Chlor-4-fluorbenzoylchlorid ist über eine mehrstufige Synthese zugänglich. Gemäß Diep et al., J. Chem. Soc. [1963], 2784-2787 setzt man zunächst ortho-Chlorfluorbenzol mit Acetylchlorid in Gegenwart großer Mengen von Aluminiumchlorid in Anwesenheit von Schwefelkohlenstoff, Methylenchlorid oder Tetrachlorethylen um, zersetzt das Reaktionsgemisch mit verdünnter Salzsäure, wäscht die organische Phase mit wäßrigem Natriumhydroxid, anschließend mit Wasser, trocknet über Natriumsulfat, dampft das Lösungsmittel ab und destilliert den Rückstand fraktioniert bei reduziertem Druck. Auf diese Weise erhält man 3-Chlor-4-fluor-acetophenon in einer Ausbeute von etwa 80 %.

Anschließend setzt man das 3-Chlor-4-fluoracetophenon mit einer wäßrigen Lösung von Natriumhypobromit um, behandelt die wäßrige Phase mit Natriumhydrogensulfit, säuert mit Salzsäure an und kristallisiert das ausgefallene Produkt aus Benzol um. Man erhält 3-Chlor-4-fluorbenzoesäure in einer Ausbeute von 74 %.

Aus der 3-Chlor-4-fluorbenzoesäure läßt sich gemäß DE 29 14 915, Seite 20, Zeilen 28 bis 35 durch Umsetzung mit Thionylchlorid in Gegenwart kleiner Mengen von Pyridin das 3-Chlor-4-fluorbenzoylchlorid herstellen.

Dieses von ortho-Chlorfluorbenzol ausgehende Verfahren weist eine Reihe von Nachteilen auf. Zum einen erfordert es vergleichsweise viele einzelne Arbeitsschritte und geht zum anderen von einem relativ schwer zugänglichen und teuren Ausgangsmaterial, nämlich ortho-Fluorchlorbenzol aus. Darüber hinaus erfordert die Friedel-Crafts-Acetylierung den Einsatz großer Mengen Aluminiumchlorid und lange Reaktionszeiten (50 Stunden). Sie wird zudem in Gegenwart problematischer Lösungsmittel, beispielsweise Schwefelkohlenstoff, durchgeführt. In der Stufe der Herstellung von 3-Chlor-4-fluorbenzoylchlorid beträgt die Ausbeute lediglich 57 %. Bezogen auf ortho-Fluorchlorbenzol erhält man 3-Chlor-4-fluorbenzoylchlorid in einer Ausbeute von lediglich 33,7 %.

Es ist aus der Publikation von E.Hope und G.C. Riley, J.Chem. Soc. [1922] 121, Seiten 2510-2527 bekannt, daß Benzoylchlorid mit Chlor in Gegenwart von Eisen(III)chlorid zu 3-Chlorbenzoylchlorid umgesetzt werden kann. Wie aus den Analysenergebnissen auf Seite 2525 hervorgeht, bilden sich neben 76 % monochlorierter Verbindungen nicht unerhebliche Mengen dichlorierte Verbindungen, nämlich 5 %. Der Anteil nicht umgesetzten Benzoylchlorids beträgt 13,5 %. Das 3-Chlorbenzoylchlorid ist allerdings durch erhebliche Mengen unerwünschter isomerer verunreinigt. Ihr Anteil beträgt nicht weniger als 16,5 % (14,5 % 2-Chlorbenzoylchlorid und 2 % 4-Chlorbenzoylchlorid).

Im Hinblick auf den vorstehend geschilderten Sachverhalt bestand daher die Aufgabe, ein Verfahren zur Herstellung von 3-Chlor-4-fluorbenzoylchlorid bereitzustellen, das die zuvor beschriebenen Nachteile vermeidet und sich auf einfache Weise auch technisch realisieren läßt. Zudem soll es das gewünschte Produkt in guten Ausbeuten und sehr guter Reinheit zugänglich machen.

Diese Aufgabe wird gelöst durch ein Verfahren zur Herstellung von 3-Chlor-4-fluorbenzoylchlorid. Es ist dadurch gekennzeichnet, daß man 4-Fluorbenzaldehyd mit einem Chlorierungsmittel in Anwesenheit eines Radikalstarters in Anwesenheit oder Abwesenheit eines Lösungsmittels bei -20 bis 200°C zu 4-Fluorbenzoylchlorid umsetzt und das 4-Fluorbenzoylchlorid mit einem Chlorierungsmittel in Anwesenheit eines Chlorierungskatalysators in Anwesenheit oder Abwesenheit eines Solvens bei -20 bis 200°C zu 3-Chlor-4-fluorbenzoylchlorid umsetzt.

Die Vorteile des erfindungsgemäßen Verfahrens bestehen unter anderem darin, daß man zum einen von einem in technischen Mengen zugänglichen Ausgangsmaterial, nämlich 4-Fluorbenzaldehyd, ausgehen kann und zudem auf die Verwendung eines Friedel-Crafts-Katalysators wie Aluminiumchlorid, der üblicherweise in sehr großen Mengen eingesetzt werden muß, verzichtet werden kann. Darüber hinaus läßt sich die Umsetzung mit vergleichsweise wenigen Arbeitsschritten mit vergleichsweise kurzen Reaktionszeiten durchführen.

Es ist ferner als sehr überraschend anzusehen, daß die erfindungsgemäße Umsetzung von 4-Fluorbenzoylchlorid mit einem Chlorierungsmittel zu 3-Chlor-4-fluorbenzoylchlorid nicht nur mit guter Ausbeute sondern auch mit sehr hoher Selektivität erfolgt. Im Hinblick auf die Ergebnisse der Chlorierung von Benzoylchlorid wäre nicht nur eine Bildung von dichlorierten Produkten sondern auch von monochlorierten isomeren 4-Fluorbenzoylchloriden in nennenswertem Umfang zu erwarten gewesen. Dies ist jedoch völlig unerwartet nicht der Fall. Isomeres 2-Chlor-4-fluorbenzoylchlorid bildet sich lediglich in einer Menge von etwa 1 % und Dichlorfluorbenzoylchloride entstehen in einer Menge unter 0,5 %.

Man setzt üblicherweise 4-Fluorbenzaldehyd und das Chlorierungsmittel im Molverhältnis 1:(0,2 bis 1,5), insbesondere 1:(0,25 bis 1), bevorzugt 1:(0,5 bis 1) ein.

Als Chlorierungsmittel kann man Chlor oder ein Chlor abgebendes Agens einsetzen. Geeignet als Chlorierungsmittel sind Chlor, Sulfurylchlorid, Thionylchlorid, Phosphortrichlorid, Phosphorpentachlorid, Antimonpentachlorid, Jodtrichlorid, Schwefeldichlorid, Dischwefeldichlorid, Mangantetrachlorid oder ein Gemisch derselben.

Man setzt 4-Fluorbenzaldehyd insbesondere mit Chlor, Sulfurylchlorid, Thionylchlorid oder einem Gemisch derselben als Chlorierungsmittel um.

Es hat sich als besonders günstig erwiesen. 4-Fluorbenzaldehyd mit Chlor als Chlorierungsmittel umzusetzen.

Die Umsetzung von 4-Fluorbenzaldehyd mit dem Chlorierungsmittel, erfolgt nach Art einer radikalischen Chlorierung, wobei die Anwesenheit eines Radikalstarters nützlich ist. Üblicherweise verwendet man ein Peroxid, eine Azoverbindung, Licht einer Wellenlänge von 200 bis 400 nm einzeln oder in Kombination miteinander als Radikalstarter. Es ist bekannt, daß organische Peroxide und organische Azoverbindungen unter dem Einfluß von Wärme und/oder Licht in Radikale zerfallen, die die radikalische Chlorierung initiieren.

Beispiele für geeignete Peroxide oder Azoverbindungen sind Ethylmethylketonperoxid, 4,4'-Azobis-(4-cyanpentansäure), 1,1'-Azobis-(cyclohexancarbonitril), α,α'-Azo(isobutyronitril), α,α'-Azodiisobutyroamidin-dihydrochlorid, tert.-Butylhydroperoxid, tert.-Butyl-trimethylsilylperoxid, Cumolhydroperoxid, Dibenzoylperoxid, Di-tert.-butylperoxid, Lauroylperoxid und/oder Perbenzoesäure-tert.-butylester, insbesondere α,α'-Azo(isobutyronitril), tert.-Butylhydroperoxid, Dibenzoylperoxid und/oder Lauroylperoxid. Licht einer Wellenlänge von 200 bis 400, insbesondere 250 bis 300 nm hat sich als besonders geeignet erwiesen.

Nach einer besonders bevorzugten Variante verwendet man Azobisisobuttersäurenitril (α,α'-Azo(isobutyronitril)) als Radikalstarter.

Man setzt den Radikalstarter (Peroxid und/oder Azoverbindung) üblicherweise in einer Menge von 0,1 bis 10, insbesondere 0,5 bis 5, bevorzugt 0,7 bis 2 Mol-%, bezogen auf 4-Fluorbenzaldehyd ein.

Die Umsetzung von 4-Fluorbenzaldehyd mit dem Chlorierungsmittel erfolgt in Anwesenheit oder Abwesenheit eines Lösungsmittels. Man kann als Lösungsmittel einen chlorierten aliphatischen oder aromatischen Kohlenwasserstoff, eine aliphatische Carbonsäure mit 1 bis 6 Kohlenstoffatomen einsetzen. Beispiele für geeignete Lösungsmittel sind 1,2-Dichlorethan, Methylchlorid, Methylenchlorid, Chloroform, Tetrachlorkohlenstoff, Trichlorbenzol, Eisessig, Acetanhydrid, insbesondere 1,2,4-Trichlorbenzol, Methylchlorid, Eisessig, Acetanhydrid.

In einer Vielzahl von Fällen hat es sich als ausreichend erwiesen, 4-Fluorbenzaldehyd mit dem Chlorierungsmittel bei 0 bis 120°C, insbesondere bei 40 bis 100°C umzusetzen.

Nach einer besonderen Ausführungsform des erfindungsgemäßen Verfahrens setzt man 4-Fluorbenzaldehyd mit dem Chlorierungsmittel in Abwesenheit eines Lösungsmittels um. Diese Variante läßt sich recht einfach durchführen und ist daher von besonderem Interesse.

Das durch die Umsetzung von 4-Fluorbenzaldehyd mit dem Chlorierungsmittel gebildete 4-Fluorbenzoylchlorid wird in einem nachfolgenden Reaktionsschritt zu 3-Chlor-4-fluorbenzoylchlorid umgesetzt.

Üblicherweise isoliert man das 4-Fluorbenzoylchlorid, beispielsweise durch Destillation, und setzt es anschließend mit einem Chlorierungsmittel zu 3-Chlor-4-fluorbenzoylchlorid um.

Es ist allerdings auch möglich, das 4-Fluorbenzoylchlorid nicht zu isolieren, sondern es beispielsweise in der Form, wie es bei der Umsetzung von 4-Fluorbenzaldehyd mit dem Chlorierungsmittel anfällt, weiter zu verarbeiten.

Man setzt üblicherweise 4-Fluorbenzoylchlorid und das Chlorierungsmittel im Molverhältnis 1:(0,2 bis 1,5), insbesondere 1:(0,25 bis 1), bevorzugt 1:(0,5 bis 1) um.

Man setzt 4-Fluorbenzoylchlorid mit einem der vorstehend bei der Umsetzung von 4-Fluorbenzaldehyd genannten Chlorierungsmittel oder einem Gemisch derselben um. Hierbei können die in den beiden Chlorierungsschritten verwendeten Chlorierungsmittel gleich oder verschieden sein. Das Verfahren gestaltet sich einfach, wenn in beiden Chlorierungsschritten dasselbe Chlorierungsmittel verwendet wird.

In einer Vielzahl von Fällen hat es sich als günstig erwiesen, 4-Fluorbenzoylchlorid mit Chlor, Sulfurylchlorid, Thionylchlorid oder einem Gemisch derselben als Chlorierungsmittel umzusetzen.

Nach einer besonderen Ausführungsform des erfindungsgemäßen Verfahrens setzt man 4-Fluorbenzoylchlorid mit Chlor um.

Die Umsetzung des 4-Fluorbenzoylchlorids wird in Anwesenheit eines üblichen Chlorierungskatalysators durchgeführt. Man kann als Chlorierungskatalysator beispielsweise Jod, Jodtrichlorid, Jodpentachlorid, Eisen, Eisen(II)chlorid, Eisen(III)chlorid, Aluminiumchlorid, Dischwefeldichlorid, Antimontrichlorid, Antimonpentachlorid oder ein Gemisch derselben verwenden.

Für eine Vielzahl von Fällen hat es sich als günstig erwiesen, Jod, Jodtrichlorid, Eisen(III)chlorid, Dischwefeldichlorid oder ein Gemisch derselben als Chlorierungskatalysator einzusetzen. Nach einer besonderen Ausführungsform verwendet man Eisen(III)chlorid als Chlorierungskatalysator.

Der Chlorierungskatalysator wird in katalytischen Mengen, d.h. nicht in stöchiometrischer Menge, bezogen auf die umzusetzende Verbindung eingesetzt.

Üblicherweise setzt man 0,001 bis 0,05, insbesondere 0,002 bis 0,03 Mol Chlorierungskatalysator je Mol zu chlorierende Verbindung ein.

Als Solvens setzt man eines der vorstehend bei der Umsetzung von 4-Fluorbenzaldehyd mit dem Chlorierungsmittel genannten Lösungsmittel oder ein Gemisch derselben ein. Die in den beiden Chlorierungsschritten verwendeten Lösungsmittel können gleich oder verschieden sein. Das Verfahren gestaltet sich besonders einfach, wenn die in den beiden Chlorierungsstufen verwendeten Lösungsmittel gleich sind.

In einer Vielzahl von Fällen hat es sich als ausreichend erwiesen, 4-Fluorbenzoylchlorid mit dem Chlorierungsmittel bei 30 bis 150°C, insbesondere bei 70 bis 130°C umzusetzen.

Nach einer besonderen Ausführungsform des erfindungsgemäßen Verfahrens setzt man 4-Fluorbenzoylchlorid mit dem Chlorierungsmittel in Abwesenheit eines Lösungsmittels um. Diese Variante läßt sich recht einfach durchführen und ist daher von besonderem Interesse.

Das Verfahren läßt sich diskontinuierlich oder kontinuierlich durchführen. Es läßt sich unter Normaldruck, Überdruck oder Unterdruck durchführen.

Die nachfolgenden Beispiele beschreiben das Verfahren näher, ohne es zu beschränken.

### Experimenteller Teil:

### Beispiel 1:

### Herstellung von 4-Fluorbenzoylchlorid

In einem 1I-Vierhalskolben werden unter Schutzgas 620 g 4-Fluorbenzaldehyd (5 mol tel quel, Reingehalt > 99 GC[a/a]) vorgelegt und mit 10 g Azobis(isobuttersäurenitril) unter Rühren versetzt. Anschließend wird auf 60°C Innentemperatur geheizt und es werden innerhalb von 7,5 Stunden insgesamt 354 g Chlor eingeleitet. Das Chlor wird in einer Menge von 15 Liter/Stunde zugesetzt. Anschließend wird nicht umgesetztes Chlor mit Stickstoff ausgeblasen, das Reaktionsgemisch auf Raumtemperatur abgekühlt. Das Fortschreiten der Umsetzung wird mittels gaschromatographischer Analyse (GC) kontrolliert.

Man erhält ein Produkt folgender Zusammensetzung:

| | |
|---|---|
| GC-Analyse | 10,7 % (a/a) 4-Fluorbenzaldehyd |
| | 78,8 % (a/a) 4-Fluorbenzoylchlorid |
| | 8,3 % (a/a) 4,4'-Difluorbenzil |
| | |

| | |
|---|---|
| Selektivität | 88,2 % |
| Umsatz | 89,3 % |
| Ausbeute | 78,7 % d.Th. |

Anschließend erfolgt die Produktdestillation i.Vak. über eine Füllkörperkolonne (Höhe 120 cm, Füllung Sulzer CY) mit einem Rücklaufverhältnis von 15:1, wobei 4-Fluorbenzoylchlorid bei 48 mbar bei konstant 107°C übergeht. Das fraktionierte 4-Fluorbenzoylchlorid weist eine Reinheit von > 99,8 % (a/a) auf und wird in der Folgestufe mittels Kernchlorierung umgesetzt.

### Beispiel 2:

### Herstellung von 3-Chlor-4-fluorbenzoylchlorid

845 g 4-Fluorbenzoylchlorid (5,3 mol tel quel, Reingehalt: 99,8 % (a/a)) werden in einer Chlorierungsapparatur unter Schutzgas mit 50,3 g Eisen(III)chlorid-Katalysator versetzt und auf 90°C Innentemperatur geheizt. Bei dieser Temperatur werden insgesamt 370 g Chlorgas (5,2 mol) kontinuierlich eingeleitet, wobei der Chlorgasstrom auf einen Wert von ca. 12 Liter/Std. eingestellt wird.
Die abschließende GC-Analyse des Reaktionsproduktes liefert folgendes Ergebnis:

| | |
|---|---|
| 23,8 % (a/a) | 4-Fluorbenzoylchlorid |
| 1,0 % (a/a) | 2-Chlor-4-fluorbenzoylchlorid |
| 71,8 % (a/a) | 3-Chlor-4-fluorbenzoylchlorid |
| < 0,5 % (a/a) | Dichlorfluorbenzoylchlorid |

| | |
|---|---|
| Selektivität | 94,2 % |
| Umsatz | 76,2 % |
| Ausbeute | 71,8 % d.Th. |

Anschließend wird das Reaktionsgemisch im Vakuum (24 mbar) destillativ vom Eisen-Katalysator abgetrennt. Das dabei gewonnene Rohdestillat wird über eine Drehbandkolonne fraktioniert destilliert, wobei die Verunreinigungen entfernt werden.
Man erhält ein 3-Chlor-4-fluorbenzoylchlorid folgender Reinheit (GC-Analyse):
0,16 % (a/a) 2-Chlor-4-fluorbenzoylchlorid
99,6 % (a/a) 3-Chlor-4-fluorbenzoylchlorid (Sdp.: 104,7°C/21 mbar, Schmelzpunkt: 28°C)

Zur zusätzlichen Charakterisierung wird das 3-Chlor-4-fluorbenzoylchlorid in die 3-Chlor-4-fluorbenzoesäure umgewandelt.

### Herstellung von 3-Chlor-4-fluorbenzoesäure:

2 g 3-Chlor-4-fluorbenzoylchlorid (Reingehalt 99,6 % GC [a/a]) werden vorsichtig in 4 g 50 %ige Natronlauge und 20 g Wasser bei 60°C hydrolysiert. Anschließend wird die Lösung noch 2 Stunden bei Raumtemperatur nachgerührt. Sodann wird mit konz. Salzsäure auf pH = 1 gestellt, die ausgefallene 3-Chlor-4-fluorbenzoesäure abgesaugt und mit Wasser gewaschen und getrocknet. Man erhält so 1,68 g 3-Chlor-4-fluorbenzoesäure in Form eines weißen Pulvers.

| | |
|---|---|
| Ausbeute | 92,8 % d.Th. |
| Schmelzpunkt | 136°C (Lit: 136°C) |

## Patentansprüche

1. Verfahren zur Herstellung von 3-Chlor-4-fluorbenzoylchlorid, **dadurch gekennzeichnet, daß** man 4-Fluorbenzaldehyd mit einem Chlorierungsmittel in Anwesenheit eines Radikalstarters in Anwesenheit oder Abwesenheit eines Lösungsmittels bei -20 bis 200°C zu 4-Fluorbenzoylchlorid umsetzt und das 4-Fluorbenzoylchlorid mit einem Chlorierungsmittel in Anwesenheit eines Chlorierungskatalysators in Anwesenheit oder Abwesenheit eines Solvens bei -20 bis 200°C zu 3-Chlor-4-fluorbenzoylchlorid umsetzt.

2. Verfahren nach Anspruch 1, **dadurch gekennzeichnet, daß** man 4-Fluorbenzaldehyd und das Chlorierungsmittel im Molverhältnis 1:(0,2 bis 1,5), insbesondere 1:(0,25 bis 1), bevorzugt 1:(0,5 bis 1) umsetzt.

3. Verfahren nach Anspruch 1 oder 2, **dadurch gekennzeichnet, daß** man 4-Fluorbenzaldehyd mit Chlor, Sulfurylchlorid, Thionylchlorid, Phosphortrichlorid, Phosphorpentachlorid, Antimonpentachlorid, Jodtrichlorid, Schwefeldichlorid, Dischwefeldichlorid, Mangantetrachlorid oder einem Gemisch derselben als Chlorierungsmittel umsetzt.

4. Verfahren nach einem oder mehreren der Ansprüche 1 bis 3, **dadurch gekennzeichnet, daß** man 4-Fluorbenzaldehyd mit Chlor umsetzt.

5. Verfahren nach einem oder mehreren der Ansprüche 1 bis 4, **dadurch gekennzeichnet, daß** man ein Peroxid, eine Azoverbindung, Licht einer Wellenlänge von 200 bis 400 nm einzeln oder in Kombination miteinander als Radikalstarter verwendet.

6. Verfahren nach einem oder mehreren der Ansprüche 1 bis 5, **dadurch gekennzeichnet, daß** man Azobisisobuttersäurenitril als Radikalstarter verwendet.

7. Verfahren nach einem oder mehreren der Ansprüche 1 bis 6, **dadurch gekennzeichnet, daß** man als Lösungsmittel einen chlorierten, aliphatischen oder aromatischen Kohlenwasserstoff, eine aliphatische Carbonsäure mit 1 bis 6 Kohlenstoffatomen einsetzt.

8. Verfahren nach einem oder mehreren der Ansprüche 1 bis 6, **dadurch gekennzeichnet, daß** man 4-Fluorbenzaldehyd mit dem Chlorierungsmittel in Abwesenheit eines Lösungsmittels umsetzt.

9. Verfahren nach einem oder mehreren der Ansprüche 1 bis 8, **dadurch gekennzeichnet, daß** man 4-Fluorbenzoylchlorid und das Chlorierungsmittel im Molverhältnis 1:(0,2 bis 1,5), insbesondere 1:(0,25 bis 1), bevorzugt 1:(0,5 bis 1) umsetzt.

10. Verfahren nach einem oder mehreren der Ansprüche 1 bis 9, **dadurch gekennzeichnet, daß** man 4-Fluorbenzoylchlorid mit einem der in Anspruch 3 genannten Chlorierungsmittel oder einem Gemisch derselben umsetzt.

11. Verfahren nach einem oder mehreren der Ansprüche 1 bis 10, **dadurch gekennzeichnet, daß** man als Chlorierungskatalysator Jod, Jodtrichlorid, Jodpentachlorid, Eisen, Eisen(II)chlorid, Eisen(III)chlorid, Aluminiumchlorid, Dischwefeldichlorid, Antimontrichlorid, Antimonpentachlorid oder ein Gemisch derselben verwendet.

12. Verfahren nach einem oder mehreren der Ansprüche 1 bis 11, **dadurch gekennzeichnet, daß** man als Solvens eines der in Anspruch 7 genannten Lösungsmittel oder ein Gemisch derselben einsetzt.

13. Verfahren nach einem oder mehreren der Ansprüche 1 bis 11, **dadurch gekennzeichnet, daß** man 4-Fluorbenzoylchlorid mit dem Chlorierungsmittel in Abwesenheit eines Solvens umsetzt.

## Claims

1. A process for the preparation of 3-chloro-4-fluorobenzoyl chloride, which comprises reacting 4-fluorobenzaldehyde with a chlorinating agent in the presence of a free-radical initiator in the presence or absence of a solvent at from -20 to 200°C to give 4-fluorobenzoyl chloride, and reacting the 4-fluorobenzoyl chloride with a chlorinating agent in the presence of a chlorination catalyst in the presence or absence of a solvent at from -20 to 200°C to give 3-chloro-4-fluorobenzoyl chloride.

2. The process as claimed in claim 1, wherein 4-fluorobenzaldehyde and the chlorinating agent are reacted in the molar ratio 1:(0.2 to 1.5), in particular 1:(0.25 to 1), preferably 1:(0.5 to 1).

3. The process as claimed in claim 1 or 2, wherein 4-fluorobenzaldehyde is reacted with chlorine, sulfuryl chloride, thionyl chloride, phosphorus trichloride, phosphorus pentachloride, antimony pentachloride, iodine trichloride, sulfur dichloride, disulfur dichloride, manganese tetrachloride or a mixture thereof as chlorinating agent.

4. The process as claimed in one or more of claims 1 to 3, wherein 4-fluorobenzaldehyde is reacted with chlorine.

5. The process as claimed in one or more of claims 1 to 4, wherein a peroxide, an azo compound or light having a wavelength of from 200 to 400 nm are used individually or in combination with one another as free-radical initiator.

6. The process as claimed in one or more of claims 1 to 5, wherein azobisisobutyronitrile is used as free-radical initiator.

7. The process as claimed in one or more of claims 1 to 6, wherein the solvent used is a chlorinated, aliphatic or aromatic hydrocarbon or an aliphatic carboxylic acid having from 1 to 6 carbon atoms.

8. The process as claimed in one or more of claims 1 to 6, wherein 4-fluorobenzaldehyde is reacted with the chlorinating agent in the absence of a solvent.

9. The process as claimed in one or more of claims 1 to 8, wherein 4-fluorobenzoyl chloride and the chlorinating agent are reacted in the molar ratio 1:(0.2 to 1.5), in particular 1:(0.25 to 1), preferably 1:(0.5 to 1).

10. The process as claimed in one or more of claims 1 to 9, wherein 4-fluorobenzoyl chloride is reacted with one of the chlorinating agents mentioned in claim 3 or a mixture thereof.

11. The process as claimed in one or more of claims 1 to 10, wherein the chlorination catalyst used is iodine, iodine trichloride, iodine pentachloride, iron, iron(II) chloride, iron(III) chloride, aluminum chloride, disulfur dichloride, antimony trichloride, antimony pentachloride or a mixture thereof.

12. The process as claimed in one or more of claims 1 to 11, wherein the solvent used is one of the solvents mentioned in claim 7 or a mixture thereof.

13. The process as claimed in one or more of claims 1 to 11, wherein 4-fluorobenzoyl chloride is reacted with the chlorinating agent in the absence of a solvent.

## Revendications

1. Procédé pour la préparation de chlorure de 3-chloro-4-fluorobenzoyle, **caractérisé en ce qu'**on fait réagir le 4-fluorobenzaldéhyde avec un agent de chloration en présence d'un amorceur radicaalire, en présence ou l'absence d'un solvant, à une température de -20 à 200°C, pour obtenir le chlorure de 4-fluorobenzoyle et on fait réagir le chlorure de 4-fluorobenzoyle avec un agent de chloration en présence d'un catalyseur de chloration en présence ou l'absence d'un solvant, à une température de -20 à 200°C, pour obtenir le chlorure de 3-chloro-4-fluorobenzoyle.

2. Procédé selon la revendication 1, **caractérisé en ce qu'**on fait réagir le 4-fluorobenzaldéhyde et l'agent de chloration dans un rapport molaire de 1:(0,2 à 1,5), notamment de 1:(0,25 à 1), de préférence de 1:(0,5 à 1).

3. Procédé selon la revendication 1 ou 2, **caractérisé en ce qu'**on fait réagir le 4-fluorobenzaldéhyde avec le chlore, le chlorure de sulfuryle, le chlorure de thionyle, le trichlorure de phosphore, le pentachlorure de phosphore, le pentachlorure d'antimoine, le trichlorure d'iode, le dichlorure de soufre, le dichlorure de disulfure, le tétrachlorure de manganèse ou un mélange de ces composés en tant qu'agent de chloration.

4. Procédé selon une ou plusieurs des revendications 1 à 3, **caractérisé en ce qu'**on fait réagir le 4-fluorobenzaldéhyde avec le chlore.

5. Procédé selon une ou plusieurs des revendications 1 à 4, **caractérisé en ce qu'**on utilise, en tant qu'amorceur de radicaux, un peroxyde, un composé azoïque, la lumière d'une longueur d'onde de 200 à 400 nm seuls ou une combinaison entre eux.

6. Procédé selon une ou plusieurs des revendications 1 à 5, **caractérisé en ce qu'**on utilise l'azobisbutyronitrile en tant qu'amorceur radicalaire.

7. Procédé selon une ou plusieurs des revendications 1 à 6, **caractérisé en ce qu'**on utilise comme solvant un hydrocarbure, un acide carboxylique aliphatique présentant 1 à 6 atomes de carbone.

8. Procédé selon une ou plusieurs des revendications 1 à 6, **caractérisé en ce qu'**on fait réagir le 4-fluorobenzaldéhyde avec l'agent de chloration en l'absence d'un solvant.

9. Procédé selon une ou plusieurs des revendications 1 à 8, **caractérisé en ce qu'**on fait réagir le chlorure de 4-fluorobenzoyle et l'agent de chloration dans un rapport molaire de 1:(0,2 à 1,5), en particulier de 1:(0,25 à 1), de préférence de 1:(0,5 à 1).

10. Procédé selon une ou plusieurs des revendications 1 à 9, **caractérisé en ce qu'**on fait réagir le chlorure de 4-fluorobenzoyle avec un agent de chloration indiqué à la revendication 3 ou un mélange de ces agents.

11. Procédé selon une ou plusieurs des revendications 1 à 10, **caractérisé en ce qu'**on utilise, en tant que catalyseur de chloration, l'iode, le trichlorure d'iode, le pentachlorure d'iode, le fer, le chlorure de fer(II), le chlorure de fer(III), le chlorure d'aluminium, le dichlorure de disulfure, le trichlorure d'antimoine, le pentachlorure d'antimoine ou un mélange de ces composés.

12. Procédé selon une ou plusieurs des revendications 1 à 11, **caractérisé en ce qu'**on utilise comme solvant un des solvants cités à la revendication 7 ou un mélange de ces solvants.

13. Procédé selon une ou plusieurs des revendications 1 à 11, **caractérisé en ce qu'**on fait réagir le chlorure de 4-fluorobenzoyle avec l'agent de chloration en l'absence d'un solvant.
